# EUROPEAN PATENT SPECIFICATION

(11) **EP 0 910 367 B1**
(45) Date of publication and mention of the grant of the patent: **15.10.2003**
(21) Application number: 97927104.6
(22) Date of filing: 02.06.1997
(51) Int. Cl.: A61K 31/355

(54) **USE OF VITAMIN E ACETATE**
VERWENDUNG VON VITAMINE E ACETAT
UTILISATION DE LA VITAMINE E ACETATE

(30) Priority: 31.05.1996 IT MI961121
(43) Date of publication of application: 28.04.1999
(60) Divisional application: 03012503.3
(73) Proprietor: BIO.LO.GA. S.r.l., 31015 Conegliano (TV) (IT)
(72) Inventor: PANIN, Giorgio, I-45100 Rovigo (IT)
(74) Representative: Ferreccio, Rinaldo
(86) International application number: EP9702856
(87) International publication number: WO97045098

(56) References cited:
- EP-A- 0 231 777
- EP-A- 0 319 963
- EP-A- 0 467 218
- EP-A- 0 539 215
- EP-A- 0 579 079
- WO-A-93/03720
- CH-A- 670 951
- DE-A- 4 125 871
- DE-A- 4 328 871
- DE-A- 4 431 251
- GB-A- 2 268 402
- US-A- 4 144 325
- US-A- 4 857 321
- US-A- 5 153 230
- US-A- 5 425 954
- US-A- 5 593 682
- US-A- 5 645 826
- DATABASE WPI Section Ch, Week 9715 Derwent Publications Ltd., London, GB; Class B02, AN 97-161412 XP002066612 & JP 09 030 964 A (SEKISUI CHEM IND CO LTD)
- DATABASE WPI Section Ch, Week 9726 Derwent Publications Ltd., London, GB; Class B05, AN 97-285134 XP002066613 & JP 09 104 623 A (SEKISUI CHEM IND CO LTD)
- DATABASE WPI Section Ch, Week 9715 Derwent Publications Ltd., London, GB; Class B02, AN 97-161413 XP002066614 & JP 09 030 965 A (SEKISUI CHEM IND CO LTD)
- DATABASE WPI Section Ch, Week 9736 Derwent Publications Ltd., London, GB; Class B05, AN 97-389341 XP002066615 & JP 09 169 643 A (SEKISUI CHEM IND CO LTD)
- PATENT ABSTRACTS OF JAPAN vol. 013, no. 414 (C-635), 13 September 1989 & JP 01 151516 A (SHIONOGI & CO LTD), 14 June 1989,

## Description

In general, the present invention relates to the use of vitamin E acetate, for the manufacture of a medicament.

Vitamin E and its derivatives are substances which, by virtue of their presumed properties as antioxidants and removers of free radicals, are widely used in the cosmetics industry in the preparation of formulations for combatting or preventing unsightly skin conditions.

For these reasons, vitamin E and its derivatives are often present in cosmetic formulations, in concentrations variable from 0.5 to 10%, as "active" components with antioxidant and anti-ageing effects.

In fact, the antioxidant and anti-ageing effect of vitamin E at skin level has never been scientifically demonstrated.

Compositions for topical use containing up to 20% by weight (preferably up to 10%) of vitamin E in combination with other active ingredients, emulsifiers and excipients are described in application EP-A-0 158 090. In this application, it is maintained that the aforesaid compositions are useful in the treatment of eczema, skin inflammation and irritation, skin allergies, skin pigmentation, etc., although experimental support is supplied solely for the application of ointments containing 8% of non-esterified vitamin E for the prevention of erythema due to exposure to the sun or to radiation.

WO 93/03720 discloses a method of treating sunburns in human or animal skin, comprising the application to the sunburned area of a topical composition comprising as active ingredient a tocopherol ester, e.g tocopherol acetate. DE 4 431 251 discloses a cream containing in a tocopherol acetate for treating atopic eczema and pseriasis.

GB 2 268 402 describes topical compositions for the treatment of acne, damaged skin, spots or wrinkles, comprising tretinoin and vitamin E

DE 4 125 871 discloses dermatological compositions including, among the other components, tocopherol or derivatives thereof.

The present invention relates to the use of vitamin E acetate for the manufacture of a medicament consisting of vitamin E for the topical treatment of a condition chosen among proctitis, anal rhagades, moist anus, haemorrhoïds and atrophic rhinitis.

The term vitamin E is intended to mean both alphatocopherol in racemic form (d, l) and the individual enantiomers and mixtures thereof.

The application of pure vitamin E acetate, which is very easy per se, can be further facilitated and rendered as uniform as possible by spray dispensers.

In this case, the vitamin E acetate is dissolved in volatile diluents which evaporate instantaneously or within a few seconds after the application of the spray, leaving a film solely of vitamin E acetate

Preferably, spray dispensing systems which do not use organic volatile or gaseous compounds as propellants are used, such as, for example, the EP SPRAY SYSTEMS® from the Swiss firm EP SPRAY SYSTEM S.A., which uses compressed air.

### Anal rhagades

The positive effect achieved by the topical application of pure vitamin E acetate is particularly noteworthy in the case of anal rhagades which represent a pathology which is difficult to treat and which, in the current state of knowledge, does not benefit from any medical treatment.

Patients suffering from single anal rhagades with or without inflammation of the mucous membrane of the edges or of the haemorrhoidal plexus were treated with pure vitamin E acetate applied topically.

Two applications (0.2-0.4 ml at a time) per day were carried out for one or two months.

In all of the patients, healing of the subjective symptoms relating to the rhagades and objective disappearance of the lesion were achieved.

Complete healing was confirmed upon programmed checking three months after the suspension of the treatment with pure vitamin E acetate.

### Haemorrhoids

Patients suffering from symptomatic haemorrhoids (heavy feeling and irritation, itching, pain and bleeding) were also treated with pure vitamin E acetate with two applications (0.2-0.4 ml at a time) per day for 15 days. In all of the patients, the beneficial effect on the symptoms was immediate with disappearance or marked attenuation thereof from the very first application and cessation of bleeding after treatment for a few days. A significant reduction (about 50%) in the size of the haemorrhoids was also observed.

The treatment, which continued for several months in all of the patients, maintained the reduction in the volume of the haemorrhoids by 2° to 3° (prolapsed) without reappearance of bleeding of the haemorrhoids and almost complete control of the symptoms was achieved.

### Moist anus

Positive effects connected with the topical application of vitamin E acetate were also found in the pathological condition known as moist anus.

Moist anus is a pathological condition characterized by a secerning, inflamed anus with associated cracking of the skin-mucous membrane and intense pain (such as to interfere with regular night-time sleep) which may arise after haemorrhoidectomy or owing to reduced tonicity of the anal sphincter or allergic diathesis thereof.

Vitamin E acetate was used (0.2-0.4 ml 3 times per day) in 3 patients (aged 30-45 years) suffering from moist anus. In all cases disappearance of the cracking and of the inflammation was achieved after treatment for one month and the beneficial effect on the symptoms (pain, burning sensation) was immediate from the very first day of treatment.

### Aspecific proctitis

Pure vitamin E acetate was used for one month (2 applications per day, each of 0.2-0.4 ml) in 20 patients with aspecific symptomatic proctitis present for a period varying from a few days to a few years. The symptoms were characterized by itching, irritation, a heavy feeling in the anus and sometimes slight bleeding upon defecation associated with exacerbation of the symptoms. The use of vitamin E acetate brought about a rapid benefit in all cases (from the very first application) with marked attenuation of the symptoms and of the proctitis (70%) or even complete disappearance thereof with clinical healing of the proctitis in a certain percentage of cases (30%).

In 30% of cases in which healing was achieved, the treatment with vitamin E acetate was suspended after the initial month with no reappearance of the symptoms or proctitis in subsequent months.

In the remaining 70% of cases, the clinical and symptomatological benefit was maintained only by continuation of the treatment with vitamin E acetate beyond the initial month with cessation of the benefit upon suspension of the treatment.

### Atrophic rhinitis

Finally, a beneficial effect was found in connection with the application of vitamin E acetate to the nasal mucous membrane in 5 cases of atrophic rhinitis.

Atrophic rhinitis is associated with a particular dryness of the nasal mucous membrane which is extremely irritating for the patient and may lead to ulceration of the mucous membrane.

Vitamin E acetate was applied (0.2-0.4 ml twice a day) for one month to the nasal mucous membrane of 5 patients suffering from atrophic rhinitis with disappearance of the nasal dryness from the very first applications. It can probably be assumed that continuous use of vitamin E acetate would prevent the ulceration which occurs with the development of the disease.

## Claims

1. Use of vitamin E acetate for the manufacture of a medicament consisting of vitamin E acetate for the topical treatment of a condition chosen among proctitis, anal rhagades, moist anus and haemorrhoids.

2. Use according to claim 1, wherein the medicament is in a package together with a notice indicating that the medicament may be used for the topical treatment of proctitis, anal rhagades, moist anus and haemorrhoids.

3. Use of vitamin E acetate for the manufacture of a medicament consisting of vitamin E acetate for the topical treatment of atrophic rhinitis.

4. Use according to claim 3 wherein the medicament is in a package together with a notice indicating that the medicament may be used for the topical treatment of atrophic rhinitis.

5. Use according to any one of claims 1 to 4, wherein the medicament is applied by spray dispensers.

## Patentansprüche

1. Verwendung von Vitamin-E-Acetat zur Herstellung eines Medikaments, bestehend aus Vitamin-E-Acetat, für die topische Behandlung einer Erkrankung, die aus der Gruppe Proktitis, Analfissuren, feuchter Anus und Hämorrhoiden ausgewählt ist.

2. Verwendung nach Anspruch 1, wobei das Medikament im Paket zusammen mit einem Hinweis vorliegt, der angibt, dass das Medikament für die topische Behandlung von Proktitis-, Analfissuren, feuchtem Anus und Hämorrhoiden verwendet werden kann.

3. Verwendung von Vitamin-E-Acetat zur Herstellung eines Medikaments, bestehend aus Vitamin-E-Acetat, für die topische Behandlung von atrophischer Rhinitis.

4. Verwendung nach Anspruch 3, wobei das Medikament im Paket zusammen mit einem Hinweis vorliegt, der angibt, dass das Medikament für die topische Behandlung von atrophischer Rhinitis verwendet werden kann.

5. Verwendung nach einem der Ansprüche 1 bis 4, wobei das Medikament mittels Sprühverteiler angewendet wird.

## Revendications

1. Utilisation de la vitamine E acétate pour la fabrication d'un médicament consistant en vitamine E acétate pour le traitement topique d'un état choisi parmi la rectite, la rhagade anale, l'anus suintant et les hémorroïdes.

2. Utilisation selon la revendication 1, dans laquelle le médicament se trouve dans un conditionnement avec une notice indiquant que le médicament peut être utilisé pour le traitement topique de la rectite, de la rhagade anale, de l'anus suintant et des hémorroïdes.

3. Utilisation de la vitamine E acétate pour la fabrication d'un médicament consistant en vitamine E acétate pour le traitement topique de la rhinite atrophique.

4. Utilisation selon la revendication 3, dans laquelle le médicament se trouve dans un conditionnement avec une notice indiquant que le médicament peut être utilisé pour le traitement topique de la rhinite atrophique.

5. Utilisation selon l'une quelconque des revendications 1 à 4, dans laquelle le médicament est appliqué par des pulvérisateurs de délivrance.
